# EUROPEAN PATENT SPECIFICATION

(11) **EP 1 324 802 B1**
(45) Date of publication and mention of the grant of the patent: **15.11.2006**
(21) Application number: 01979245.6
(22) Date of filing: 11.09.2001
(51) Int. Cl.: A61M 31/00

(54) **MYOGENIC CELL TRANSFER CATHETER**
KATHETER FÜR DEN MYOGENEN ZELLTRANSFER
CATHETER DE TRANSFERT DE CELLULES MYOGENES

(30) Priority: 12.09.2000 US 231880 P
(43) Date of publication of application: 09.07.2003
(73) Proprietor: Law, Peter K., Memphis, TN 38117-7126 (US)
(72) Inventor: Law, Peter K., Memphis, TN 38117-7126 (US)
(74) Representative: Hinkelmann, Klaus
(86) International application number: PCT/US2001/028712
(87) International publication number: WO 2002/028470

(56) References cited:
- WO-A-98/22021
- WO-A-99/04850
- DE-U1- 29 609 350
- US-A- 5 130 141
- US-A- 5 733 727
- US-A- 5 916 194
- US-A- 6 015 671
- US-B1- 6 200 303
- US-B1- 6 224 566
- US-B1- 6 261 832

## Description

The invention relates to materials for repairing and augmenting tissues in a host. More specifically the invention relates to cardiovascular catheters for automatically injecting large quantities of cells such as myogenic cells for repairing and augmenting muscle and other tissues.

### BACKGROUND OF THE INVENTION

Cell therapy such as myogenic cell therapy promises to revolutionize the treatment of serious muscular diseases such as muscular dystrophy and heart disease as pioneered by Law et al. (See Gene Ther. Mol. Biol. pp. 345-363 (March 1998), Cell Transplantation 6: 95-100 (1997) and U.S. Pat. No. 5,130,141). According to this cell biology approach to tissue repair and augmentation, myogenic cells are cultured and then introduced into a target muscle tissue such as striated muscle or heart muscle. Within the tissue, the introduced cells fuse with pre-existing muscle cells, transferring the normal genome in their nuclei to effect genetic repair. The introduced cells also fuse among themselves to form normal cells to replenish cells that have degenerated. Myogenic cell therapy is very promising and promises to revolutionize the treatment of heart disease, with enormous consequences to the society. A major impediment to the widespread use of myogenic cell therapy however is the need to implant very high numbers of cells in order to overcome the initial cell rejection, presumably due to the action of scavenging macrophages, as reviewed by Law *et al,* in *Gene Ther. Mol. Biol.* (*Id.* p. 351).

The cell number problem is a major impediment to progress in this field. That is, there is no good tool and procedure for implanting large numbers of cells to region(s) of body structures such as muscle that are degenerated or diseased and which can benefit from the cell treatment. Accordingly, new techniques and tools for implanting large numbers of cells are key to success and can unlock the potential of cell therapy to treat diseases such as heart disease and other muscular disorders.

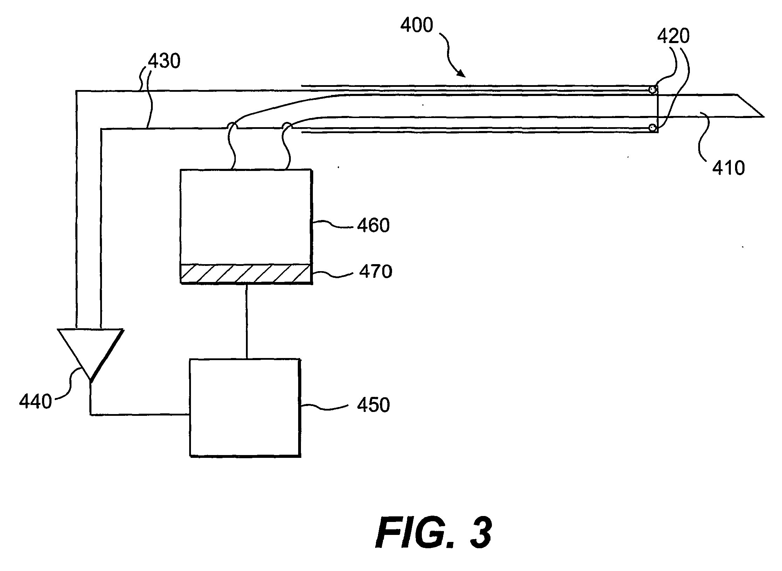

Unfortunately previously known catheters and methods are not suitable for targeted delivery of cells, and particularly for the large numbers required for cell transplant therapy. For example, U.S. Nos. 5,865,787 and 5,634,899 describe delivery of a drug using electric current/voltage control. Such delivery by electric current is not compatible with living cells. As another example U.S. No. 6,056,969 describes implantation of drug in pellet form by piercing the heart muscle wall (see Fig. 3 and Fig. 4). However, such pellets are incompatible with cells that are delivered in a suspension and that need to remain mobile, particularly for the large volumes needed for cell therapy.

A second problem often encountered is that there is no sufficiently reliable way to implant material into a moving target such as the heart wall from the inside of the heart while the heart is beating. Present methods are hampered by, for example, the need to pace or interrupt the heart beat as injection is not carried out easily on a moving target.

Present techniques for implanting medicaments to body tissues generally rely on injection directly into body tissue such as intraperitoneal injection, intravenous injection and intramuscular injection as, for example explored in U.S. Pat. No. 5,130,141 for delivery of myogenic cells to muscles of normal and of diseased hosts. Heretofore crude attempts to implant cells into the heart primarily have emulated the simple injection of drugs, using syringe injection by hand during open heart surgery, as for example, taught by Field in U.S. Pat. No. 5,602,301. Such techniques are highly unsuitable in the real world situation, however where opening a chest cavity is undesirable, and wherein hand injection with a needle gives rise to errors that affect a vital organ. The heart muscle presents a particular challenge to hand manipulated procedures because this muscle is constantly beating and presents a moving target.

Less invasive catheters and techniques have been developed for administration of drugs into structures such as the heart, as exemplified by U.S. Nos. 6,059,770 ("Catheter Providing Intraluminal Access"), 5,865,787 ("Simultaneous Cardiac Pacing and Local Drug Delivery"), 5,634,899 ("Simultaneous Cardiac Pacing and Local Drug Delivery Method'), 6,059,969 ("System and Methods for Local Delivery of an Agent") and 6,016,437 ("Catheter Probe System with Inflatable Soft Shafts"). These devices provide a means to enter a body space such as an artery or interior of the heart from a remote entry site. Catheters have been used for various purposes, but have not been used successfully for cell implantation.

A particular need of cell therapy in the context of heart repair is to detect which portion(s) of the heart need to be treated and receive cells. In the case of the heart muscle a diseased part will exhibit electrical silence, diminished electrical activity, and/or abnormal electrocardiogram. Catheters have been developed that can map local cardiac signals with electrodes on their ring and tip surfaces, as reviewed in U.S. Pat. No. 5,450,846. Some of those catheters have been used for making an intracardiac electrogram, as described in U.S. Pat. No. 5,156,151. However, the art has not progressed sufficiently to conveniently identify specific small spots throughout the entire myocardium that are degenerated or weak in a manner that the spot can be repaired via cell therapy, particularly during the same procedure. Thus, an obstacle to cell therapy of myocardial tissue is that a suitable diagnostic catheter for that purpose is not available.

### SUMMARY OF THE INVENTION

In contrast with limitations of the art, preferred embodiments of the invention provide new tools that can 1) access the interior of a body or body structure such as the heart via a percutaneous and endovascular route, 2) electrically detect even minute degenerative areas of a body surface such as the endomyocardium, 3) insert a needle of predetermined diameter (gauge) and length, preferably 2 to 8 mm long, more preferably 3 to 5 mm long, preferably 29 gauge to 10 gauge, more preferably 28 gauge to 18 gauge, even more preferably 27 gauge to 22 gauge, preferably diagonally into the structure, at and/or around the degenerative area, 4) injecting a predetermined number of cells (such as myoblasts) through the needle, preferably 10 to 800 million cells, more preferably 25 to 200 million cells and even more preferably 50 to 100 million cells, 5) through an electrically coupled feed-back circuit and mechanical system, 6) within a predetermined period of time, preferably less than one second and 7) without the need of extensive opening of the body through, for example, an open chest or open heart surgery.

In one embodiment, the invention provides an apparatus for a method of delivering myogenic cells into a degenerated or weak muscle in a host, comprising the step of placing a catheter within the host, the catheter comprising a sensor on its tip to detect degenerated or weak muscle tissue on contact; and expelling the myogenic cells through a needle in the tip of the catheter into and around the degenerative or weak muscle areas upon sensing contact with degenerated or weak muscle tissue. In another embodiment the invention is a catheter for injection of myogenic cells into a target portion of a heart comprising a flexible needle within the catheter having a bore of twenty-two to twenty seven gauge; a tip on the catheter that is tapered to allow close contact with the inside wall of the heart and that contains at least one electrode to monitor an electrical signal of the heart muscle portion that it contacts; a tip on the needle that protrudes from the catheter tip no more than 5 millimeters to prevent perforation of the heart; a needle fluidically connected to a source of myogenic cells; and an electrical feedback circuit that transduces any aberrant electrical signal from the catheter tip into automatically injecting the myogenic cells into a target heart material within one second.

Another embodiment of the apparatus of the invention is useful for a method for treating angina pectoris in a patient with a catheter having a reservoir, and a tip, the tip comprising two or more electrodes and at least one needle for delivering myogenic cells, comprising the steps of providing myogenic cells for use with the catheter; placing the myogenic cells in the reservoir and placing the catheter inside the patient and/or into the heart of the patient; detecting one or more degenerative portions on the inside surface of the heart tissue; and automatically injecting myogenic cells from the needle into and around the portion of the degenerative or weak heart tissue.

In yet another embodiment the invention is a catheter for detecting and treating local areas of muscle degeneration and weakness in a patient, comprising a long body that can be inserted into the patient; a tip at the end of the long body; at least three electrodes on the tip; and a needle for injecting cells into the muscle, the needle having a point; wherein the three electrodes form a flat surface on the end of the tip, the long axis of the needle is positioned between the three electrodes and is perpendicular to the flat surface.

In a further embodiment the invention is an apparatus for treating a body tissue by implantation of cells into selected parts of an organ of a patient in vivo comprising a catheter, a retracted needle and at least two electrodes in its tip; a reservoir of cells to be implanted; an actuator for moving the needle from the catheter and into a selected part of the organ; an actuator for forcibly expelling cells through the needle and into the selected part of the organ; and a controller that activates the actuators wherein the controller monitors electrical signals obtained by the electrodes, compares those signals with stored or calculated information and activates needle movement and cell movement upon detection of proximity of the selected part, whether normal or abnormal, of the organ to the catheter.

### DESCRIPTION OF THE FIGURES

Figure 1 is a diagram of a catheter tip having two electrodes, one needle and a solenoid driver according to an embodiment of the invention.
Figure 2 is a diagram of a catheter tip having three electrodes and an extended needle according to an embodiment of the invention.
Figure 3 is a diagram showing a placement of a catheter inside a heart according to an embodiment of the invention.
Figure 4 is a diagram of a catheter and controller system according to an embodiment of the invention.

### DETAILED DESCRIPTION OF THE INVENTION

The inventor overcame the problem of delivering large numbers of cells to appropriate (degenerative and/or weak) locations in a moving heart with new automated catheters that automatically deliver large amounts of cells via locational targeting, optional controlled injection depth and timing to damaged sites of a heart. An automated catheter according to the invention has an electrode-feedback system that detects damaged tissue on, for example, heart muscle and automatically and rapidly injects large quantities of myogenic cells into the detected tissue in real time. That is, the catheter system detects a suitable site at virtually the same time as it implants cells to improve that site, thus overcoming the severe problem that the heart is moving during the procedure. In preferred embodiments the catheter tip has a needle that is triggered to protrude by a defined distance that is long enough to safely inject cells into the myocardial tissue but not too long as to puncture the outer wall of the heart. Preferably the needle is completely retracted into a sheath during insertion of the catheter into a patient. In an embodiment the system minimizes operator error by virtue of an electronic-mechanical coupled system that electronically detects one or more weak or degenerative spots on the myocardium and automatically injects a controlled amount of myogenic cells into and/or around that same site, all without human intervention.

An important feature of the catheter for heart repair is that the injection be carried out rapidly while the heart is beating. During anesthesia, the heart may contract approximately once per second. The catheter solves the timing problem by detecting contact with a weak or degenerated spot and then quickly injecting cells automatically. Preferably this time is less than one second, more preferably less than 0.7 seconds, most preferably in less than 0.5 seconds.

In a preferred embodiment of the inventive apparatus, the catheter: (1) accesses the interior of the heart via a percutaneous and endovascular route without necessitating opening of the chest cavity or an open heart surgery; (2) electrically detects even minute degenerative areas of the endomyocardium; 3) inserts a needle of predetermined diameter (gauge) and length, preferably 2 to 8 mm long, more preferably 3 to 5 mm long, preferably 29 gauge to 10 gauge, more preferably 28 gauge to 18 gauge, even more preferably 27 gauge to 22 gauge, preferably diagonally into the myocardium, at and/or around the degenerative area, 4) injecting a predetermined number of cells (myoblasts) through the needle, preferably 10 to 800 million cells, more preferably 25 to 200 million cells and even more preferably 50 to 100 million cells, 5) through an electrically coupled feed-back circuit and mechanical system, 6) within a predetermined period of time, preferably less than one second and 7) without the need for an open chest or an open heart surgery.

### Catheter Structure

A variety of catheters and catheter control systems for their use to treat muscle degeneration and weakness in a host (eg. complications of heart disease) are contemplated according to the invention. In each instance, cells are loaded into a reservoir and are injected into the muscle under timing control by an electronic feedback circuit. In most contemplated uses the muscle is a heart muscle and the catheter is maneuvered within the inside of the heart by a medical practitioner. The feedback circuit is one link in a control system that comprises the catheter itself with electrodes in the catheter tip and a controller that contains electrical circuits for detecting, modifying and analyzing signals obtained from the electrodes. The feedback circuit further comprises an actuating mechanism for expelling a controlled volume or amount of myogenic cell suspension and optionally, to thrust a recessed myogenic cell delivery needle out of the catheter tip into the selected muscle tissue surface.

Previously known catheters are commonly used in percutaneous procedures to, for example, dilate stenosed blood vessels or arteries, to deliver medicines, to deliver electric shocks and the like. Aspects of such catheters may be combined with the present invention. A wide variety of catheters having one or more useful characteristics in that regard are known, as exemplified by wire catheters illustrated generally in U.S. Pat. No. 4,323,071; fixed wire catheters of the type illustrated in U.S. Pat. No. 4,582,181; rapid exchange catheters as illustrated in U.S. Pat. No. 4,762,129 and other catheters for drug delivery and mapping as illustrated in U.S. Pat. Nos. 5,865,787; 6,016,437; 5,941,845; 5,941,845; 5,634,899, 6,011,889, 5,450,846; 6,059,759; and 5,002,067.

In practice, a catheter according to embodiments of the invention comprises proximal and distal ends (with respect to placement of the medical practitioner user), a suspension of myogenic cells, and one or more needles that are fluidically connected to a source of medicinal material such as a suspension of myogenic cells. Preferably, the catheter is soft so as not to cause hemorrhage of the body part such as the heart. Accordingly, the catheter body may comprise pliable plastic. In a preferred embodiment a needle is pushed out of the catheter tip between 3-12 mm and preferably 5-10 mm and more preferably, for regular non-degenerative adult hearts, 6-8 mm from the surface of the catheter. The needle preferably exits from the very distal end surface of the tip. In some embodiments, the needle may exit from a corner of the tip end, or even from the side of the tip. During contraction of the heart and/or movement of the catheter by the operator, the tip presses against the wall of the heart and the needle enters the heart muscle by the defined distance. In preferred embodiments the needle exits preferably within a short time interval and the cells are expelled through the needle into the myocardium, all within less than the time of a single heart beat.

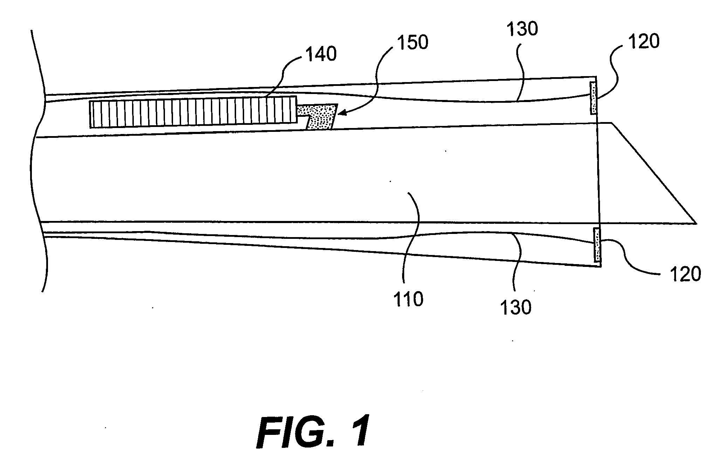

In one embodiment the needle normally remains within the tip of the distal end of the catheter and moves out of the catheter during injection of cells. Figure 1 shows an embodiment where needle 110 is within catheter 100 or protruding a small distance. Electrode surfaces 120 send electric signals via wires 130 to a controller (not shown). The controller activates solenoid 140 to automatically push cells out of needle 110.

The catheter tip preferably is tapered to allow insertion into or penetration of a body cavity such as a femoral artery or coronary artery. The catheter body should be flexible enough (as described in many of the cited patents) to allow the tip to be manipulated to the desired site such as the interior of the heart. The catheter preferably has two or more electrodes on a flat surface of the tip. The centers of the electrodes are axially displaced 50 to 2000 µm from each other, preferably 100 to 1000 µm from each other and most preferably between 150 to 500 µm from each other. Preferably the electrodes are within the plane of a flat surface that terminates at the catheter tip, as shown in Figure 3. In some embodiments one or more electrodes are present on the side of the catheter tip as shown, for example in U.S. Pat. No. 5,450,846.

The electrodes are electrically connected to conductive wires in the catheter, printed conductive (and insulated) lines on the catheter surface or other conductive material that extend from the tip to the proximal end of the catheter where they are connected electrically to a control system. Although direct conductivity is preferred, it will be recognized that other means of transferring information is possible, including for example, use of a miniature radio transmitter from inside the catheter to a receiver outside the host (patient). The exterior surface of the catheter may comprise a coating of heparin, hirudin, antibiotics or the like or other substance chosen for biocompatibility with the host.

In an embodiment the catheter further contains an electro-mechanical device for pushing a needle within the catheter tip into muscle tissue after sensing of mechanical contact between the catheter tip and the muscle tissue and activation via the controller. In preferred embodiments the needle in the tip of the catheter extends throughout the catheter and is connected to a reservoir of myogenic cells outside of the catheter.

### Cells Administered by the Catheter

A wide range of cells are useful for the invention, as described for example in U.S. Pat. Nos. 5,130,141 and 6,015,671. In each case, the cells are provided in aqueous suspension with optional polymer for mechanical stabilization and/or to improve subsequent attachment to the site of delivery. Examples of suspension media and suitable optional polymers are provided in U.S. Pat. No. 5,567,612.

Specialized cell media may be used in conjunction with the catheter. In one such embodiment, a polymer is included to minimize leakage and/or movement of cells after injection into the myocardium. Polymers preferred for this use include for example, those described in U.S. Pat. No. 6,051,648. Polymers and binding agents that adhere to muscle cells and myocardium tissue are particularly preferred, and may be used in combination with other polymers or substances that bind and hold myogenic cells in place for a period of time before dissolution or release of the binding agents. The concentration and average sizes of polymers used for these purposes will be readily determined through routine experimentation and chosen to minimize viscosity of the injected cell suspension. In one embodiment freshly oxygenated red cells are included in the media to maintain aerobic respiration of myogenic cells that are loaded and remain within the catheter for a period of time prior to their injection.

Myogenic cells according to the invention pass through the needle in the catheter tip and are injected into a suitable myocardial (or other tissue) inner surface by operation of the catheter and its control system. In some embodiments the cells are loaded into a reservoir of the lumen catheter shortly before use of the catheter. The reservoir is fluidically connected to the injection needle. In other embodiments the cells are within a reservoir attached to the catheter and pass through the catheter length to the needle shortly before or during use of the catheter. In the latter case, shortly before use the space within the needle optionally may be purged of air by pulling body fluid from inside the patient into the needle and from there down to the proximal end of the needle. Then the body fluid is expelled by forcing in the other direction back out of the needle opening.

### Needles used by the Catheter

A catheter according to embodiments of the invention contains preferably one needle but may have more than one for delivering cells to the target muscle. The needle preferably is between 19 to 30 gauge, more preferably between 21-28 gauge and most preferably between 22 to 27 gauge. The needle has an internal bore with no constriction, thus allowing smooth cell movement. The needle preferably is within the tip of the catheter either on the leading edge or on the side of the tip within 10 mm from the leading edge. The tip preferably protrudes between 3 and 12 mm and more preferably between 5 and 8 mm from the surface of the catheter. In one embodiment the needle is retracted within the catheter tip during insertion of the catheter into a patient and then is pushed out after entering the target organ such as the interior of a heart. In another embodiment the needle does not protrude beyond the tip under normal (non-injecting) conditions and is activated by a controller, which pushes the needle outside of the tip through a distance that preferably is between 3 and 12 mm and more preferably between 5 and 8 mm. In preferred embodiments the tip is flat and, as manipulated by the user, can sit flush on the surface of the myocardium.

In a preferred embodiment the needle comprises metal and acts as a common electrode to form one or more detecting circuits with one or more other electrodes at the catheter tip. The needle-electrode monitors electrical activity to detect the condition of a muscle, particularly heart muscle that the tip contacts or is adjacent to. In another embodiment, two needles are present and arranged on opposite sides of the tip. Both needles can be used as electrodes. In the latter case, a controller may select one needle or the other to deliver cells.

In preferred embodiments the bore or the inside of the needle is polished and made smooth by rubbing with a tungsten wire. In this case, tungsten wire is preferably threaded inside and used to polish the inside surface(s) of the needle until they become smooth. This embodiment limits cell lysis during injection of cells through the needle.

The needle, in one embodiment exists within the catheter lumen and is pushed out along the axis of the catheter when activated as shown in Figure 1. In another embodiment the needle is perpendicular to a plate or flat surface of the catheter tip and gets pushed out of that surface at an oblique angle or perpendicular with respect to the catheter axis, when activated. For both embodiments, actuation of needle movement optionally may be performed by a solenoid or piezo electric device.

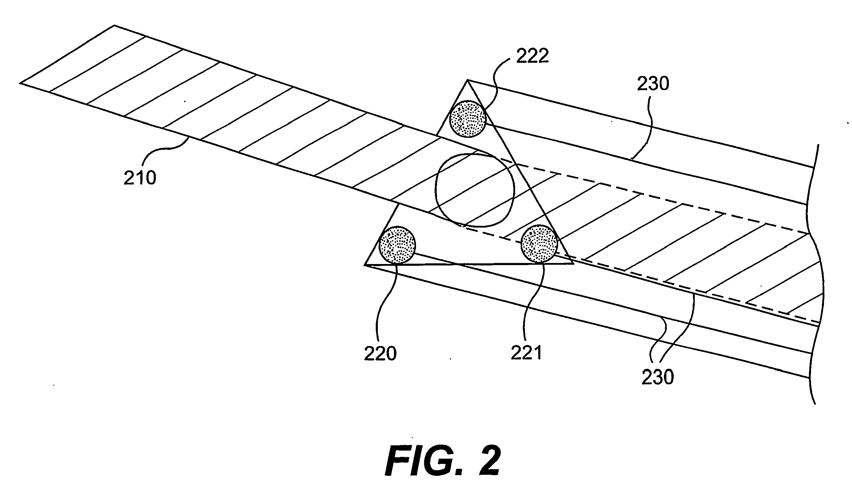

In a preferred embodiment the needle permanently is extended from the catheter surface as shown in Figure 2. During use, the catheter is maneuvered within the heart and allows the needle to penetrate the myocardium. The electrical activity within the muscle is monitored by circuits outside the catheter to determine whether that portion of the muscle is degenerative or weak (decrease in or absence of normal electrical activity). If the electrical activity (or lack thereof) indicates a denerative or weak spot then myogenic cells are automatically expelled out of the needle into the muscle.

### Electrodes used in the Catheter

Electrodes have been used in prior art catheters to detect special locations of the heart, such as, for example, the AV junction as described in U.S. Pat. No. 6,059,726 and for mapping fine detail in the heart, including pairs of sensors as described, for example by U.S. Pat. Nos. 5,865,787 and 5,450,846. Sensor materials such as carbon, silver, silver chloride plated silver and platinum are well known in the art and are suitable. Such electrodes are connected to wires or other conductors within and/or on the outer surface of the catheter. The wires connect to circuitry and create signals that can be analyzed by a computer.

Two or more electrodes according to the invention may be used together to identify a localized area of abnormal myocardium as a site for injecting myogenic cells. The electrodes can be provided in various geometric forms, such as a flat surface perpendicular to the axis of the catheter tip, a flat orthogonal surface parallel to the axis of the catheter tip, a hollow cylinder, a sphere with a hole formed through the sphere, or a metallic bump on a flat insulating surface but preferably are two dots at corners of a flat probe tip and are used to determine when the probe tip is flush against the myocardium surface. One consideration in choosing the shape of the electrode is to confine the signal measurement area to a small region to allow detection of a localized damaged or weak muscle area. In some embodiments at least 2 electrodes are arranged on the catheter tip and an injection needle within the catheter tip is pushed out (perpendicular with) the contacted myocardium wall by a defined distance in response to signal(s) from the electrodes that indicate flush contact with a damaged myocardial surface. Optionally one electrode has a large surface area, or may be the catheter exterior surface (if electrically conductive) and is used as a ground to help provide more precise information to control circuitry about the electrical signal status of the immediate localized area.

### Control System

During use, the catheter is manipulated by the medical practitioner inside a patient and, while doing so, the electrodes participate in one or more electrical circuits to provide electrical information feedback to a "control system" about the environment of the catheter tip. The electrodes may be used to provide, for example, electrical conductivity (resistance), high impedance voltage measurements or low impedance current measurements to the control system. The electrodes are electronically connected, preferably by wires, to a sensor/controller that monitors the electrical environment of the catheter tip. The sensor/controller controls injection of the myogenic cells through a needle in the catheter tip into the myocardium and preferably is integrated with a computer that records the history of injections and triggers injection in real time according to comparison of electrode signals with stored or computed comparative information. In another embodiment the injection is controlled by hardware and the parameters to control triggering of the actuator(s) are set by characteristics of the hardware and/or by electronic controls that are set by the user.

In some embodiments two electrode pairs are connected in a circuit at any given time and either the conductivity or voltage between the electrodes is monitored. In one embodiment electrode pairs are coupled in a pair-wise fashion to differential amplifiers for generating one or more difference signals between the electrodes. The difference signals are subject to additional signal processing in conventional circuitry not explicitly depicted, such as signal conditioning and logical processing to develop a signal which ultimately is stored in a recording medium such as computer memory and also used to control injection of the myogenic cell suspension and (preferably) movement of a catheter needle as well. Further detail of the circuitry in the sensor/controller readily will be ascertained by a skilled artisan such as an electronics engineer.

The sensor/controller of the control system preferably comprises a computer, which optionally collects data in real time both from the catheter electrodes and from an imaging tool using, for example, sonography to monitor the position of the catheter. The computer may store information related to the location of each myocardium locus assayed by electrode measurements, the electrical properties, which reveal the state of that locus, and the amount of cell suspension material injected into the locus. The circuit that alters the analog signals into digital signals for entry into the computer determines when the catheter tip surface has contacted a suitable target myocardium site.

Upon contact and sensing of a desired target site, the computer or other controlling circuit quickly (preferably within 25 milliseconds, more preferably within 1 millisecond actuates efflux of myogenic cell suspension through the needle and into the target myocardium) and, according to the preferred mode where a recessed needle is used, actuates (starts) movement of a recessed needle within the catheter tip, causing it to lunge out of the catheter tip and imbed into the target myocardium. Electromechanical actuators such as a solenoid or piezo-electric device are preferred for these movements. When the myogenic cells are fluidically connected to a reservoir at the proximal end of the catheter, the actuator for pushing the cell suspension through the needle may be outside the catheter. However, the pump for expelling cells may be within the catheter. The pump may for example, be a large surface piezo electric device (more than 1 cm² surface exposed to the cell suspension) having a surface that twists in response to an electrical signal, or may be a solenoid plunger that is connected to a closed flexible reservoir that causes increased pressure in the reservoir, which is fluidically connected to the catheter needle.

Most preferably, the automatic control system monitors the heart beat and triggers an injection sequence at a suitable time between contractions to maximize the amount of time for injecting the cells. In one embodiment, a computer is used that monitors (a) the heart beat cycle and (b) detection of contact with a weak or degenerated portion of the muscle. When the timing is right (eg. beginning of a rest period of the cycle) and the tip portion containing the needle tip contacts a weak or degenerated spot, as determined by absent or abnormal electrical signal(s) then the computer triggers the needle to plunge into the heart wall and triggers the efflux of cells, all in short time that preferably is less than one second.

Due to the lag time associated with some mechanical systems, the signal for actuating efflux of cells may occur at the same time as the signal for triggering the needle to plunge into the heart. Depending on the particular mechanical mechanisms, however, triggering of cell efflux may occur slightly after triggering needle movement. In one embodiment needle movement is controlled by a first piezo electric device within the catheter and the cells are pushed out of the needle by a second pump that is connected to a catheter, and both movements are started simultaneously. In every case, it is highly preferred to coordinate the needle plunge and cell efflux with the proper timing of the heart beat and to carry out the entire sequence of events, from proximity detection of the needle tip to a weak or degenerated region of the heart, to the needle plunge and cell efflux during the time when the particular chamber of the heart being treated is not actively contracting. In another embodiment, the contraction of the heart itself is simultaneously controlled to lengthen the time for injecting the cells.

A skilled artisan can determine suitable timing for efflux of myogenic cells through the needle, and plunging a recessed needle out of the catheter tip based on known or calculated transients for the respective movements. That is, depending on the physics of the system such as the reservoir size and response to the actuator force, the needle diameter and distance, the properties (particularly viscosity) of the fluid, etc., a timing sequence can be readily determined by a skilled artisan. In one embodiment, a polymer or other thickening agent is added to the cell suspension to limit leakage of cell suspension out of the needle when not in use.

In embodiments where a cell suspension pump is located outside of the catheter, a longer pause occurs between actuation of the pump and actual efflux of material from the needle. In that embodiment the time between optional electromechanical movement of the needle into the muscle is timed to coincide with the time for efflux of cells from the needle.

While the needle is imbedded within the target myocardium, myogenic cell suspension is forced out the needle tip and deposited into the myocardium. Preferably at least 5 million cells are expelled in one injection within at least 0.1ml of volume. More preferably at least 15 million cells are expelled within at least 0.25 ml volume. Most preferably between 25 and 100 million cells are expelled in a volume of between 0.3 and 0.7 ml. In preferred embodiments a total of between 500 million cells and 5 billion cells are administered among at least 10 such injections and preferably between 25 and 60 injections. For example, an adult heart in need of repair may receive 40 0.5 ml injections for a total of 20 ml of suspension having a cell density of 100 million cells per ml. For this cell density, a 22 gauge needle is most preferred. Larger and smaller gauges are also suitable depending on the viscosity of the cell suspension that is used.

### Use of a Control System to Detect Degenerative or Weak Cardiac Tissue and to Control Injection of Myogenic Cells into that Tissue

Degenerative or weak sites of muscle tissue, such as locations of cardiac tissue that have deteriorated from heart disease, can be detected by its absent or weak electrical signals. For example, when in contact with or adjacent to normal myocardiac tissue, an electrical signal emanating from that normal tissue can be detected by a reference electrical signal, typically determined by periodicity, waveform, amplitude, and/or duration of an electrical signal measured between two electrodes on the catheter surface. In contrast, a signal from a damaged or weak target location will be absent, of lower strength (measured as a lower voltage or lower current depending on the impedance of the measurement) or of a different pattern of periodicity, waveform, amplitude and/or duration. This comparative information particularly is useful in two ways. One, the information can be used to inform the operator (and the computer) when the catheter has contacted damaged or weak tissue. Such information may be provided as an audio signal to the operator, the amplitude of which increases as the catheter moves away from healthy tissue, indicating movement to or arrival at diseased tissue. Two, the information can be used by the controller to automatically inject myogenic cells upon contact with the diseased tissue. In this context the controller preferably will have a set of stored or determined threshold values that, once exceeded indicates that the tissue next to the catheter tip is weak enough to warrant an injection of cells.

In some embodiments a combination of measurements are made. For example, resistance can be measured between electrodes to detect placement of the catheter tip on a surface. Upon flush contact, the space between the electrodes is filled by tissue and solution phase conductivity is lost. When in contact with a tissue, the conductivity is greater. This conductivity (resistance) measurement preferably is carried out using an alternating current electrical drive source (preferably above 120 Hz, more preferably above 1,000, even more preferably above 50,000 Hz) in order to distinguish that signal from the noisy electrical environment produced by the muscle tissue. A filter tuned to the frequency of the drive source preferably is used to remove the background noise components of the signal. A background control resistance is determined by conductivity between electrodes when the tip is not in contact with (in this case cardiac) tissue. Upon contact with tissue, the measured resistance increases (conductivity decreases), by a threshold amount, denoting that the space between the electrodes is occluded by the less conducting solid. The actual change in conductivity in one embodiment is provided as an audio signal to the operator to indicate when the tip is in contact with a solid (muscle) surface.

In a preferred embodiment this impedance measurement is carried out simultaneously with detection of electrical signals by the electrodes from heart tissue. Contact with heart tissue is detected by the change in impedance and a determination of whether the contacted tissue has been degenerative or became weak, is made by detecting whether the tissue produces normal healthy electrical pulses or abnormal electrical pulses (or no pulses) that indicate damaged or abnormal tissue. In a preferred embodiment both detection of electrical signals produced from the myocardium surface and the detection of impedance to determine independently, whether the tip is touching a solid surface, are carried out simultaneously with the same electrodes using one or more hardware and/or computer software filters to separate the signals from each other.

Of course, for the preferred embodiment where the needle is permanently extended by 5-8 mm from the surface of the catheter, placement of the needle into the muscle will drastically alter the conductivity and/or reception of electrical activity from that portion of that muscle, allowing an accurate determination of contact.

In another embodiment the electrodes at the catheter tip detect the position of the needle and provide positional feedback information to the control system. In a preferred embodiment, closed circuits between pairs of adjacent electrodes are established by electronic switches in the controller/sensor circuitry (the switches connect specific electrode combinations), the signals produced are buffered by operational amplifiers, and the analog signals are used, either directly to indicate a damaged target tissue, or indirectly by operation of a software program after their conversion into digital signals and input into a computer. Upon detection of a suitable target site, two events happen. One event is that the cell efflux activation (and optional needle lance activation) and are triggered. A second event is that the conductivity between the needle and one or more electrodes is monitored. As the needle moves out of the tissue, the electrical conductivity between the needle and other electrode(s) increases. This signal is used by the sensor/control circuitry and/or the computer to feedback control the cell efflux (and optional needle movement) mechanisms. In one embodiment with a retractable needle, while the piezo electric or solenoid mechanism (either inside or outside the catheter) pushes the needle out, a signal is received from the needle-electrode circuit indicating that the needle has moved by a certain amount. The signal informs the computer of the time and duration of needle movement.

In yet another embodiment of the apparatus according to the present invention, a circuit is established between the patient's body and one or more electrodes (optionally including the needle as an electrode) and used to relax the heart muscle or otherwise momentarily alter the normal rhythm of the heart to facilitate detection and/or delivery of cells and/or other material into the target tissue. That is, a circuit is established between an electrode(s) and "ground," wherein the ground is created by contacting a large surface electrode to the skin of the body. Skilled medical practitioners in this field will readily appreciate voltages and patterns needed for cardiac pacing, as exemplified in U.S. Pat. No. 5,634,899. According to one embodiment of this method, when it is difficult to make a flush contact between the catheter tip surface and a desired region of the heart, a stimulation signal may be generated from the catheter itself to the heart to alter the heart's movement and improve chances for moving the catheter to a better position.

The catheter electrode can provide two dimensional information that informs the user which direction to move the catheter, which is particularly useful for the embodiment having a retractable needle. Figure 2 shows needle 210 that extends 7mm from a surface of catheter 200. In this embodiment two or more embodiment conductivity measurements and/or two or more measurements of electrical signals produced by muscle tissue are made using electrodes 220, 221, and 222. The electrodes are connected to wires 230. Measurements can be made from the three electrode system shown in Figure 2 to provide two dimensional information about which direction to move the tip to encounter damaged myocardium. By way of example, a user may manipulate the catheter tip shown in Figure 2 to sit flush on the surface of a myocardium. The control system then compares conductivity (or voltage produced by myocardium itself) in a circuit formed between electrode 220 and electrode 221, in a circuit formed between electrode 220 and electrode 222, and in a circuit formed between electrode 222 and electrode 221. When a region of cardiac muscle that has more damage is located immediately to the top and right of the line formed between electrode 222 and 221 (i.e. away from electrode 220) then the electrical signal between electrodes 222 and 221 is smaller of the three signals (or the abnormal characteristic of the signal between electrodes 222 and 221 is greater). The control system monitors and compares signals, both temporally and (preferably) spacially to obtain this information. Of course, other combinations and signal comparisons can be made and will be apparent to a skilled artisan. Preferably, a data base is obtained in a model system such as a pig heart system to generate representative voltages, impedances and conductivities for a given multiple electrode catheter system.

### Use of the System for Myogenic Cell Therapy

The system described herein can be used for injecting cells into a muscle lining any body space such as found in stomach, intestine, bladder, arteries, heart. Most preferred uses are for within the heart although both epicardial tissue can be treated as well as endocardial tissue. In preferred endocardial embodiments the medical practitioner inserts the catheter into the femoral artery leading to the left ventricle or via the coronary artery through the aorta and into the left ventricle as depicted in Figure 3. The operator manipulates the catheter tip to contact various regions of the heart that are suspected of containing degenerative or weak tissue, such as the lateral wall of the left ventricle. The control system interfaced with the catheter monitors the electrical environment of the catheter tip and detects when the tip contacts a degenerative or weak area of the myocardial tissue. The heart is beating during this time. The control system automatically ejects cell suspension material through the needle tip and into the tissue.

The control system optionally may activate a needle to come out of the catheter tip and imbed into the endo-myocardial tissue prior to ejection of cell suspension in the embodiment that uses a retractable needle. The needle then retracts and a series of such automated injections are made at various locations.

An example of a system for cardiac therapy is shown in Figure 4. The distal end of catheter 400 is seen at the top of this figure, and has a tip to the right in which needle 410 protrudes 7 mm. Electrode surfaces 420 are arranged around needle 410 and electrically connect by wires 430 to hardware 440. Hardware 440 buffers the electrical signals and converts them into a digital form that is input into computer controller 450. Needle 410 is fluidically connected to reservoir 460, which comprises pressure activator 470. During use, computer 450 receives signals from hardware 440, compares those signals with information stored inside the computer and then turns on activator 470 upon detection of a degenerative or weak area of myocardium at the tip of catheter 400.

In preferred embodiments the injection of cells into degenerative or weak portions of myocardial tissue are carried out within a total time of less than 2 seconds, more preferably less than 1 second and most preferably less than 0.5 seconds. After making a series of injections the catheter is removed and the injected cells (if myogenic cells) gradually differentiate into heart muscle cells over time. Improved heart muscle function is detected by any of a number of techniques known to the skilled artisan.

### Use of the Invention for Myogenic Cell Therapy and Reconstruction in a Variety of Body Cavities

Most embodiments of apparatus discussed herein concern the repair and/or augmentation of heart tissue. However, it is understood that the invention also is useful for repair and augmentation of other body parts such as regular smooth muscle arteries, the stomach, the diaphram, the bladder, the liver and other tissues. For example, reconstructive surgery of bladder may, in some aspects benefit from administration of myogenic cells in a polymeric suspension to plug a gap or hole in a pre-existing muscle. In one embodiment for repair of the gastrointestinal tract, reconstruction is performed with a catheter according to the invention in a two step procedure. In the first step, myogenic cells are injected into areas that respond poorly to peristaltic signals or that poorly generate peristaltic signals. In a second step a second type of material is then injected to cover the myogenic cells of the first injection to make a patch that protects the cells from the gastrointestinal tract lining during healing. The second type of material may be administered by the same catheter or by a second device. In each case, the cells of choice are myogenic cells, which are known to fuse with other cells both in muscle tissue and fat tissue.

It is to be understood that the description, specific descriptions of embodiments and examples, while indicating exemplary embodiments, are given by way of illustration and are not intended to limit the present invention. Various changes and modifications within the present invention as defined by the claims will become apparent to the skilled artisan from the discussion, disclosure and data contained herein, and thus are considered part of the invention.

## Claims

1. An apparatus for detecting and treating local areas of muscle degeneration and weakness in a patient, comprising a reservoir (460) of myogenic cells, a catheter (100; 400) and a fluidic connection between the reservoir of myogenic cells and the catheter (100; 400), the catheter (100; 400) comprising:
a. a long body that can be inserted into the patient;
b. a tip at the end of the long body;
c. a sensor on the tip; and
d. a needle (110, 210, 410) connected to the fluidic connection, suitable for injecting the myogenic cells into the muscle, and having a point;
wherein the sensor is positioned to detect a surface at the end of the tip and the long axis of the needle (110; 210; 410) is positioned at the end of the tip.

2. An apparatus as described in claim 1, wherein the sensor comprises at least two electrodes.

3. An apparatus as described in claim 1, wherein the needle (110; 210; 410) point remains inside the catheter (100; 400) during insertion of the long body of the catheter (100; 400) into the patient and is thrust out from the tip after detection of an electrically silent or quiescent muscle surface.

4. An apparatus as described in claim 1, further comprising a reservoir actuator (470) for pushing cells out of the reservoir (460) and through the fluidic connection between the reservoir (460) and the needle (410).

5. An apparatus as described in claim 1, wherein the needle (110; 210; 410) is recessed in the tip and is pushed out of the recess by a needle actuator in response to signals obtained from the electrodes (120; 220; 221; 222).

6. An apparatus as described in claim 1, wherein the needle (110; 210; 410) for injecting cells is prepared by a method of passing a tungsten wire through the needle (110; 210; 410) multiple times.

7. An apparatus as described in claim 1, wherein the needle (110; 210; 410) is flexible and has a bore of eighteen to twenty eight gauge, and the catheter tip is tapered to allow close contact of the needle (110; 210; 410) with the inside wall of the heart.

8. An apparatus as described in claim 1, wherein the at least two electrodes (120; 220; 221; 222) are capable of sensing mechanical contact between the catheter tip and the muscle tissue such as by monitoring an electrically abnormal heart muscle inner surface.

9. An apparatus as described in claim 1, further comprising a control circuit that controls multiple injections of cells from the source of myogenic cells.

10. An apparatus as described in claim 1, wherein the needle protrudes from the catheter tip no more than 5 millimeters.

11. An apparatus as described in claim 1, further comprising a controller that monitors portions of a heart and controls delivery of the cells to a portion via the catheter (100; 400), the controller comprising.
(a) two or more monitoring circuits that monitor signals produced from electrodes in the catheter tip;
(b) a comparator (450) that comprises hardware circuitry, a computer or both, that analyzes the signals obtained from the monitoring circuits; and
(c) an activator (470) for injecting cells through the needle (110; 210; 410) and into the portion of the heart,
wherein the comparator (450) is capable of energizing the activator (470) within 50 milliseconds of receiving signals from the monitoring circuits.

12. An apparatus as described in claim 11, wherein the monitoring circuits comprise at least 3 circuits from 3 electrodes and wherein the three electrodes form a planar surface on the catheter tip.

13. An apparatus as described in claim 11, wherein the reservoir (460) further comprises freshly oxygenated red cells.

14. An apparatus as described in claim 1, wherein proximity of the catheter tip to a surface of a local area of muscle degeneration and weakness is determined by monitoring electrical resistance.

15. The apparatus of claim 1, wherein the sensor comprises at least three electrodes (120; 220; 221; 222) on the tip; and the at least three electrodes (120; 220; 221; 222) form a flat surface such that the long axis of the needle (110; 210; 410) is positioned between the at least three electrodes (120; 220; 221; 222).

16. An apparatus as described in claim 1, further comprising a signal generator, wherein the electrical signals obtained by the sensor (120; 220; 221; 222) are produced from the signal generator and electrical resistance is monitored to determine contact of the catheter (100; 400) with a surface.

17. An apparatus as described in claim 15, further comprising a control circuit that controls multiple injections of the cells.

18. An apparatus as described in claim 15, wherein the needle (110; 210; 410) protrudes from the catheter tip no more than 5 millimeters.

## Patentansprüche

1. Ein Apparat zum Nachweis und zur Behandlung von lokalen Flächen mit einer Degenerierung und Schwäche von Muskeln in einem Patienten, umfassend ein Reservoir (460) von myogenen Zellen, ein Katheter (100; 400) und eine fluide Verbindung zwischen dem Reservoir von myogenen Zellen und dem Katheter (100; 400), wobei der Katheter (100; 400) umfasst:
a) einen langen Körper, der in einen Patienten eingeführt werden kann;
b) eine Düsenspitze am Ende des langen Körpers;
c) einen Sensor auf der Düsenspitze; und
d) eine Nadel (110; 210; 410), die mit der fluiden Verbindung verbunden ist, die zur Injektion von myogenen Zellen in den Muskel geeignet ist und eine Spitze hat;
worin der Sensor derart positioniert ist, um eine Oberfläche am Ende der Düsenspitze zu detektieren und die lange Achse der Nadel (110; 210; 410) am Ende der Düsenspitze positioniert ist.

2. Ein Apparat, wie er in Anspruch 1 beschrieben ist, worin der Sensor mindestens zwei Elektroden umfasst.

3. Ein Apparat, wie er in Anspruch 1 beschrieben ist, worin die Spitze der Nadel (110; 210; 410) während der Einführung des langen Körpers des Katheters (100; 400) in den Patienten innerhalb des Katheters (100; 400) bleibt und nach der Detektion einer elektrisch ruhigen oder untätigen Muskeloberfläche aus der Düsenspitze ausgestoßen wird.

4. Ein Apparat, wie er in Anspruch 1 beschrieben ist, weiterhin umfassend einen Auslöser (470) für das Reservoir zum Herausdrücken der Zellen aus dem Reservoir (460) und durch die fluide Verbindung zwischen dem Reservoir (460) und der Nadel (410).

5. Ein Apparat, wie er in Anspruch 1 beschrieben ist, worin die Nadel (110; 210; 410) in die Eintiefung der Düsenspitze zurückgezogen ist und aus der Eintiefung durch einen Auslöser für die Nadel als Reaktion auf von den Elektroden (120; 220; 221; 222) erhaltene Signale herausgedrückt wird.

6. Ein Apparat, wie er in Anspruch 1 beschrieben ist, worin die Nadel (110; 210; 410) für die Injektion von Zellen durch eine Methode hergestellt wird, bei der ein Draht aus Wolfram mehrere Male durch die Nadel (110; 210; 410) hindurch gezogen wird.

7. Ein Apparat, wie er in Anspruch 1 beschrieben ist, worin die Nadel (110; 210; 410) flexibel ist und einen äußeren Durchmesser von achtzehn bis achtundzwanzig Gauge (G) (1,20 mm bis 0,37 mm) hat und die Düsenspitze des Katheters spitz zuläuft, um einen engen Kontakt der Nadel (110; 210; 410) mit der Innenwand des Herzen zu ermöglichen.

8. Ein Apparat, wie er in Anspruch 1 beschrieben ist, worin die mindestens zwei Elektroden (120; 220; 221; 222) in der Lage sind, einen mechanischen Kontakt zwischen der Düsenspitze des Katheters und dem Muskelgewebe zu ermöglichen wie z.B. durch Überwachung der inneren Oberfläche eines elektrisch abnormalen Herzmuskels.

9. Ein Apparat, wie er in Anspruch 1 beschrieben ist, der weiterhin einen Regelkreis umfasst, der mehrfache Injektionen von Zellen von der Quelle der myogenen Zellen regelt.

10. Ein Apparat, wie er in Anspruch 1 beschrieben ist, worin die Nadel nicht mehr als 5 Millimeter aus der Düsenspitze des Katheters herausragt.

11. Ein Apparat, wie er in Anspruch 1 beschrieben ist, der zusätzlich eine Regelung umfasst, die Teile eines Herzens überwacht und die Lieferung von Zellen zu einem Teil durch den Katheter (100; 400) regelt, wobei die Regelung umfasst:
a) zwei oder mehr Regelkreise zur Überwachung, welch die von den Elektroden in der Düsenspitze des Katheters erzeugten Signale beobachten.
b) einen Vergleicher (450), der Hardware - Schaltkreise, einen Computer oder beides umfasst, der die von den Regelkreisen zur Überwachung erhaltenen Signale analysiert; und
c) einen Aktivator (470) für die Injektion von Zellen durch die Nadel (110; 210; 410) und in den Teil des Herzen,
worin der Vergleicher (450) in der Lage ist, den Aktivator (470) innerhalb von 50 Millisekunden nach Erhalt von Signalen von den Regelkreisen zur Überwachung anzusteuern.

12. Ein Apparat, wie er in Anspruch 11 beschrieben ist, worin die Regelkreise zur Überwachung mindestens 3 Regelkreise von 3 Elektroden umfassen und worin die drei Elektroden auf der Düsenspitze des Katheters eine ebene Oberfläche bilden.

13. Ein Apparat, wie er in Anspruch 11 beschrieben ist, worin das Reservoir (460) zusätzlich frisch mit Sauerstoff angereicherte rote Zellen umfasst.

14. Ein Apparat, wie er in Anspruch 1 beschrieben ist, worin die Nähe der Düsenspitze des Katheters zu der Oberfläche einer lokalen Fläche von Degenerierung und Schwäche von Muskeln durch die Beobachtung des elektrischen Widerstands bestimmt wird.

15. Der Apparat von Anspruch 1, worin der Sensor mindestens drei Elektroden (120; 220; 221; 222) auf der Düsenspitze umfasst und die mindestens drei Elektroden (120; 220; 221; 222) eine flache Oberfläche bilden, so dass die lange Achse der Nadel (110; 210; 410) zwischen den mindestens drei Elektroden (120; 220; 221; 222) angeordnet ist.

16. Ein Apparat, wie er in Anspruch 1 beschrieben ist, der weiterhin einen Signalerzeuger umfasst, in dem die von dem Sensor (120; 220; 221; 222) erhaltenen elektrischen Signale von dem Signalerzeuger erzeugt werden und der elektrische Widerstand beobachtet wird, um den Kontakt des Katheters (100; 400) mit einer Oberfläche zu bestimmen.

17. Ein Apparat, wie er in Anspruch 15 beschrieben ist, der weiterhin einen Regelkreis umfasst, der mehrfache Injektionen der Zellen regelt.

18. Ein Apparat, wie er in Anspruch 15 beschrieben ist, worin die Nadel (110; 210; 410) nicht mehr als 5 Millimeter aus der Düsenspitze des Katheters herausragt.

## Revendications

1. Un appareil pour la détection et le traitement de la dégénérescence et de la faiblesse de zones musculaires localisées chez un patient, comprenant un réservoir (460) de cellules myogéniques, un cathéter (100; 400) et une connexion fluide entre le réservoir de cellules myogéniques et le cathéter(100; 400), le cathéter (100; 400) comprenant:
a) un corps longitudinal pouvant être inséré dans le patient;
b) une tuyère à l'extrémité du corps long;
c) un senseur sur la tuyère; et
d) une aiguille (110; 210; 410) reliée à la connexion fluide, appropriée pour l'injection des cellules myogéniques dans le muscle, et ayant une pointe;
dans lequel le senseur est positionné de manière à détecter une surface à l'extrémité de la tuyère et dans lequel l'axe longitudinal de l'aiguille (110; 210; 410) est positionné à l'extrémité de la tuyère.

2. Un appareil comme celui décrit dans la revendication 1, dans lequel le senseur comporte au moins deux électrodes.

3. Un appareil comme celui décrit dans la revendication 1, dans lequel la pointe de l'aiguille (110; 210; 410) reste à l'intérieur du cathéter (100; 400) pendant l'insertion du corps long du cathéter (100; 400) dans le patient et est projetée hors de la tuyère après la détection d'une surface musculaire électriquement silencieuse ou quiescente.

4. Un appareil comme celui décrit dans la revendication 1, comprenant de surcroît un actionneur à réservoir (470) pour pousser les cellules hors du réservoir (460) et à travers la connexion fluide entre le réservoir (460) et l'aiguille (410).

5. Un appareil comme celui décrit dans la revendication 1, dans lequel l'aiguille (110; 210; 410) est reculée dans la tuyère et avancée hors de la tuyère par un actionneur d'aiguille en réponse à des signaux obtenus à partir des électrodes (120; 220; 221; 222).

6. Un appareil comme décrit dans la revendication 1, dans lequel l'aiguille (110; 210; 410) pour l'injection des cellules est préparée selon une méthode consistant à passer le fil de tungstène plusieurs fois à travers l'aiguille (110; 210; 410).

7. Un appareil comme celui décrit dans la revendication 1, dans lequel l'aiguille (110; 210; 410) est flexible et a un diamètre de gauge 18 à 28, et le bout du cathéter est conique pour permettre un contact étroit de l'aiguille avec la paroi interne du coeur.

8. Un appareil comme celui décrit dans la revendication 1, dans lequel au moins les deux électrodes (120; 220; 221; 222) sont capables de capter un contact mécanique entre le bout du cathéter et le tissu musculaire comme celui survenant lors du monitorage de la surface interne du muscle cardiaque électriquement anormal.

9. Un appareil comme celui décrit dans la revendication 1, comprenant de surcroît un circuit de contrôle qui contrôle les injections multiples de cellules à partir de la source de cellules myogéniques.

10. Un appareil comme celui décrit dans la revendication 1, dans lequel l'aiguille ne dépasse pas de plus de 5 millimètres de la tuyère du cathéter.

11. Un appareil comme celui décrit dans la revendication 1, comprenant de surcroît un appareil de contrôle qui supervise des segments du coeur et contrôle la livraison des cellules par le cathéter (100; 400) à un segment, l'appareil de contrôle comprenant:
a) deux ou plusieurs circuits de monitorage qui supervisent des signaux produits à partir des électrodes dans la tuyère du cathéter;
b) un discriminateur (450), comprenant un réseau informatique, un ordinateur ou les deux, qui analyse les signaux obtenus à partir des circuits de monitorage; et
c) un activateur (470) pour l'injection des cellules à travers l'aiguille (110; 210; 410) et dans le segment du coeur,
dans lequel le discriminateur (450) est capable d'enclencher un activateur (470) dans les 50 millisecondes qui suivent la réception des signaux provenant des circuits de monitorage.

12. Un appareil comme celui décrit dans la revendication 11, dans lequel les circuits de monitorage comprennent au moins 3 circuits à partir de 3 électrodes et dans lequel les 3 électrodes forment une surface plane sur la tuyère du cathéter.

13. Un appareil comme celui décrit dans la revendication 11, dans lequel le réservoir (460) comprend de surcroît des cellules rouges fraîchement oxygénées.

14. Un appareil comme celui décrit dans la revendication 1, dans lequel la proximité de la tuyère de cathéter d'une surface de zone locale de dégénérescence et de faiblesse musculaire est déterminée par la résistance électrique de monitorage.

15. L'appareil de la revendication 1, dans lequel le senseur comprend au moins trios électrodes (120; 220; 221; 222) sur le bout; et qu'au moins trois électrodes (120; 220; 221; 222) forment une surface plate de sorte que l'axe longitudinal de l'aiguille (110; 210; 410) est positionné entre au moins trois électrodes (120; 220; 221; 222).

16. Un appareil comme celui décrit dans la revendication 1, comprenant de surcroît un générateur de signaux, dans lequel les signaux électriques obtenus par le senseur (120; 220; 221; 222) sont produits à partir du générateur de signaux et où la résistance électrique est contrôlée pour déterminer le contact du cathéter (100; 400) avec une surface.

17. Un appareil comme celui décrit dans la revendication 15, comprenant de surcroît un circuit de contrôle qui contrôle les injections multiples de cellules.

18. Un appareil comme celui décrit dans la revendication 15, dans lequel l'aiguille (110; 210; 410) ne dépasse pas de plus de 5 millimètres de la tuyère du cathéter.
